(19) Europäisches Patentamt / European Patent Office / Office européen des brevets

(11) **EP 4 092 413 A1**

(12) **EUROPEAN PATENT APPLICATION**
published in accordance with Art. 153(4) EPC

(43) Date of publication:
**23.11.2022 Bulletin 2022/47**

(21) Application number: **21741251.9**

(22) Date of filing: **18.01.2021**

(51) International Patent Classification (IPC):
***G01N 33/49*** (2006.01)   ***G01N 21/59*** (2006.01)

(52) Cooperative Patent Classification (CPC):
**G01N 21/59; G01N 33/49**

(86) International application number:
**PCT/JP2021/001540**

(87) International publication number:
**WO 2021/145461 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.01.2020 JP 2020006394**

(71) Applicant: **A&T Corporation**
**Fujisawa-shi, Kanagawa, 252-0816 (JP)**

(72) Inventors:
• **NAKAYAMA, Hajime**
**Fujisawa-shi, Kanagawa 252-0816 (JP)**
• **YOSHIDA, Akio**
**Fujisawa-shi, Kanagawa 252-0816 (JP)**
• **TSUBOTA, Hirokazu**
**Fujisawa-shi, Kanagawa 252-0816 (JP)**

(74) Representative: **Hepworth Browne**
**15 St. Pauls Street**
**Leeds LS1 2JG (GB)**

(54) **BLOOD-CLOTTING MEASUREMENT DEVICE, BLOOD-CLOTTING TIME MEASUREMENT METHOD, METHOD FOR DETERMINING COMPLETION OF BLOOD-CLOTTING REACTION, AND AUTOMATED CENTRIFUGAL BLOOD SEPARATOR**

(57)     It was found that when a blood collection tube (1) containing a blood specimen is irradiated with near infrared light of a specified wavelength from 1050 nanometers to 1350 nanometers, by a near infrared light source (4) and temporal variation of an intensity of light transmitted through an inside of the blood specimen and detected by an infrared camera (6) is measured, a process of a blood coagulation reaction can be observed. As a result, in addition to being able to measure the blood coagulation time, completion or incompletion of the blood coagulation reaction can be determined from an increasing or decreasing trend of the light intensity and preprocessing of centrifugation treatment can be automated.

FIG.1

**EP 4 092 413 A1**

**Description**

TECHNICAL FIELD

The present invention relates to a blood coagulation measuring device, a method of measuring blood coagulation time, a method of determining blood coagulation reaction completion, and an automated blood centrifuge device in a clinical laboratory. In particular, the invention relates to detection of a blood coagulation reaction in a blood collection tube, measuring tube, and the like, and technology for automated centrifugation treatment of a blood specimen collected in a blood collection tube and the like.

BACKGROUND ART

In the field of clinical testing such as at hospitals, a blood coagulation test is an important independent test and various blood coagulation testing devices such as mechanical devices and optical devices have been available. One example is a device named "CG02N" (manufactured by A&T Corporation).

Further, in a laboratory, depending on the purpose of the analysis, a blood specimen collected in a blood collection tube is separated into components such as hemocyte, blood clots (in the present invention, no distinction is made between a blood clot and an embolus and hereinafter, are collectively referred to as a "blood clot"), serum and blood plasma. With this aim, a device (for example, a device name "iCM" manufactured by A&T Corporation) for automated centrifugation treatment of a specimen is available and in addition to this, a system that dispenses and dilutes a predetermined amount of whole blood, serum, etc. into a secondary vessel, and automatically conveys the secondary vessel to an analyzer is available (for example, Laboratory Automation System, A&T Corporation).

Here, description is given according to centrifugation treatment of a blood specimen at a hospital laboratory. A blood coagulation accelerator such as silica particles, thrombin, etc. or a separating agent for coagulated blood, etc. is added in advance to a blood collection tube. A clinical laboratory technologist gently inverts the blood collection tube at least 5 times so as to uniformly mix a collected blood specimen and the blood coagulation accelerator without forming bubbles. After a predetermined period has elapsed and completion of the coagulation reaction has been determined visually, the blood collection tube is placed in a centrifuge and serum and hemocyte components are separated.

Nonetheless, with visual determination, there is no guarantee that the coagulation reaction has finished and, for example, when the initial inversion mixing is incomplete or when the blood is centrifuged before completion of the blood coagulation reaction, and when which a blood coagulation inhibitor is present in blood, fibrinogen may remain in the serum after centrifugation. In this instance, fibrinogen is known to be converted into a poorly-soluble fibrin monomer due to the effects of thrombin, further denaturing into a stable fibrin polymer and becoming an insoluble filamentous substance or a gel-like substance (hereinafter, this is called a "fibrin mass").

A fibrin mass causes problems in that, for example, during a pretreatment operation of dispensing the serum thereafter into a sub-specimen, the fibrin mass clogs the dispensing nozzle, whereby a specified amount of the serum cannot be collected and as a result, an abnormal test value is given. Therefore, there is a demand for centrifugation treatment to be performed after completion of the coagulation reaction is confirmed so that fibrinogen, fibrin, etc. do not remain in the serum.

On the other hand, regarding a blood coagulation measuring device, a method of measuring the blood coagulation reaction by an optical method has been proposed. For example, a halogen lamp is used as a light source and by using an optical filter, blood plasma, which is a blood component, is irradiated with multiple light beams of wavelengths in a range from 340 nanometers to 800 nanometers and the attenuation time for the amount of transmitted light is measured, whereby the coagulation time is measured (for example, refer to Patent Document 1).

Further, with respect to whole blood in a blood collection tube (in the present specification "whole blood" refers to a specimen that has not been physically treated such as by centrifugation and that is free of a substance that is intentionally added to the collected blood specimen and not originally in the blood collection tube. Therefore, even when a blood specimen contains a substance originally in the blood collection tube used during collection, such as an anticoagulant agent, a blood coagulation accelerator, an activating agent, a separating agent, the substance is treated as a substance contained in "whole blood"), for the purpose of optically detecting blood coagulation in individual blood collection tubes, a method has been disclosed in which a light source with a wavelength range of 500 nanometers to 1100 nanometers is irradiated on a blood collection tube and based on the transmitted light of a specified wavelength range 750 to 1050 nanometers, the coagulation reaction is detected using information obtained by secondary differentiation of absorbance by wavelength (for example, refer to Patent Document 2). Further, an example has been disclosed in which, for the purpose of detecting fibrin in blood plasma and to achieve increased sensitivity and to ensure dynamic range in blood coagulation analysis, multiple detectors, the light source, etc. are disposed at suitable detection angles selected according to the scattered light intensity of each measurement sample (refer to Patent Document 3).

Patent Document 1: Japanese Laid-Open Patent Publication No. 2014-173904
Patent Document 2: Japanese Laid-Open Patent Publication No. 2016-061585
Patent Document 3: Japanese Laid-Open Patent Publication No. 2015-111123

DISCLOSURE OF INVENTION

PROBLEM TO BE SOLVED BY THE INVENTION

In a conventional blood coagulation test, mainly blood plasma free of the hemocyte component is measured and visible light of 660 nanometers, infrared light of about 800 nanometers or near infrared light of 1000 nanometers or less has been used. In these wavelength regions, the effects of reflection by the surface of the blood collection tube, absorption by the hemocyte component or absorption and scattering due to aggregation of hemocyte, etc. are strong and in particular, when the tube diameter is large and the optical path length is long similarly to a blood collection tube, when whole blood is to be measured, a problem arises in that the intensity of the light that reaches a light receiving element is extremely small and detection is difficult. For these reasons, to detect the blood coagulation reaction, mainly blood plasma is measured and attempts have been made to take advantage of slight changes in the intensity of the transmitted light due to wavelength, by performing measurement by multiple wavelengths concurrently or using a light source with a continuous wavelength.

In centrifugation treatment of whole blood (in this instance, in general, a blood specimen is collected in a blood collection tube that contains a blood coagulation accelerator) for the purpose of separating the serum and a state is necessary in which the blood coagulation reaction has been completed. This is a consequence of fibrinogen and/or fibrin remaining in the obtained serum when centrifugation treatment is performed in state when the blood coagulation reaction is incomplete, whereby a fibrin mass occurs thereafter, hindering analysis work. Conventionally, in general, completion of the blood coagulation reaction is visually confirmed and determined by a clinical laboratory technologist and mechanization and automation are desirable from the viewpoint of reproducibility and certainty of the determination, the labor involved, and the like. Nonetheless, an effective technology capable of determining completion of the blood coagulation reaction while whole blood is in a blood collection tube has not conventionally existed.

Further, the technique in Patent Document 1 mainly employs a conventional technique of using light of the visible light region to measure blood plasma from which the hemocyte component has been separated, and is not applicable to whole blood. The technique further takes advantage of increases in turbidity of the measurement sample due to increased scattering resulting from fibrin aggregation when the coagulation reaction of blood components progresses, and in particular, scattering characteristics of the light are affected by wavelength and therefore, measurement and comparison is performed using 2 wavelengths to obtain the true coagulation time. Thus, a problem of redundancy of the measurement method and the measurement device arises. Further, the technique in Patent Document 2, in a wavelength region of a wide range, takes advantage of differences in the characteristics of the amount of transmitted light that occur due to the coagulation of blood components. In this method, the light source can emit a continuous wavelength, and in the light receiving unit a splitter is necessary while an arithmetic circuit for secondary differentiation of absorbance is necessary and therefore, a problem of redundancy in the configuration of the measurement device arises. Further disadvantages include scanning over a wide wavelength range for irradiation of light and measurement are necessary, and the measurement time per one measurement sample also increases. Furthermore, the purpose of the method described in Patent Document 3 is to measure scattered light of fibrin in blood plasma under optimal conditions and therefore, is not originally a technique for measuring whole blood. As described above, there is no useful optical technique for measuring transmitted light or scattered light in the analysis of blood coagulation of whole blood that is to be measured.

To solve the problems described above, an object of the present invention is to enable easy detection of the blood coagulation reaction process of whole blood in a blood collection tube. A further object is to automate the determination of the completion of the blood coagulation reaction performed by a clinical laboratory technologist and to enhance the efficiency of laboratory tests.

MEANS FOR SOLVING PROBLEM

To solve the problems above, a main means of the present invention is use of near infrared light for which the wavelength range is 1050 nanometers to 1350 nanometers. Near infrared light of this wavelength range is conventionally called "the Second Biological Window" and because transmission characteristics with respect to biological tissue are known to be favorable, transmittivity with respect to whole blood can be expected to be favorable.

A blood coagulation measuring device according to claim 1 is characterized in having: a measuring tube that holds a specimen in which whole blood and a blood coagulation accelerator are mixed with each other; a thermostatic device capable of holding the measuring tube at a constant temperature; a measurement light source capable of irradiating the measuring tube with a near infrared light of a specified wavelength range belonging to a range from 1050 nanometers to 1350 nanometers; a light receiving circuit unit that continuously measures an amount of light of the specified wavelength range transmitted through an inside of a specimen in the measuring tube; and an arithmetic processing device that sets a starting point to be when the blood coagulation accelerator is mixed and measures a time period when the amount of the light begins to increase until the amount of the light changes to decreasing. In the present invention, "light transmitted through an inside of a specimen" is defined as light that is incident to and passes through the inside of a specimen and is emitted externally, irrespective of direction after emission. Further, the light needs not be light in a single direction after emission and may be a combination of light in different directions. Moreover, in the present invention "transmitted light" is defined to include, in addition to "direct transmitted light" that passes straight through the inside of a specimen along the optical axis of the measurement light source and is emitted, light (diffused transmitted light) that is input to the same light receiving circuit unit as the direct transmitted light and that is affected by a substance in the specimen, resulting in the direction of the light being changed but the amount of change being slight.

The blood coagulation measuring device according to claim 2 is characterized in that, as a particularly favorable wavelength range, a wavelength of the specified wavelength range is in a range from 1250 nanometers to 1300 nanometers.

The blood coagulation measuring device according to claim 3 is characterized in that a peak wavelength of the measurement light source is a LED light source of a single wavelength in a range from 1050 nanometers to 1350 nanometers. The light source not only reduces device size, but also has an advantage of less noise such as scattered light and stray light because excess light is not emitted due to the half-width being short.

A method of determining blood coagulation reaction completion according to claim 4, characterized in having: a process of irradiating a specimen obtained by mixing a blood coagulation accelerator and whole blood collected in a vessel, the specimen being irradiated with a near infrared light from a measurement light source; a process of measuring an intensity of light transmitted through an inside of the specimen, the intensity of the light being an amount of light that is of a specified wavelength range belonging to wavelengths from 1050 nanometers to 1350 nanometers; and a determining process of determining that a blood coagulation reaction is finished or not finished, based on information regarding an increase or decrease of the amount of the light during an arbitrary measurement. As a result, information of increases/decreases of the specimen at an arbitrary time after collection is measured, whereby completion/incompletion of the blood coagulation reaction can be determined.

The method of determining blood coagulation reaction completion according to claim 5 is characterized in that a wavelength of the specified wavelength range is present in a range from 1250 nanometers to 1300 nanometers.

The method of determining blood coagulation reaction completion according to claim 6 is characterized in that a peak wavelength of the measurement light source is a LED light source of a single-wavelength in a range from 1050 nanometers to 1350 nanometers.

The method of determining blood coagulation reaction completion according to claim 7 is characterized in that the determining process includes determining that the coagulation reaction is in progress when the amount of the light is increasing, and determining that the coagulation reaction is finished when the amount of the light is decreasing.

The method of determining blood coagulation reaction completion according to claim 8 is characterized in that the determining process is a process of concurrently measuring transmitted light and scattered light intensities.

A method of determining blood coagulation reaction completion according to claim 9 is characterized in having: a process of irradiating a specimen obtained by mixing a blood coagulation accelerator and whole blood collected in a cylindrical vessel, the specimen being irradiated with a near infrared light from a measurement light source; a process of measuring an intensity of a transmitted light transmitted through an inside of the specimen, the intensity of the transmitted light being an amount of the transmitted light that is of a specified wavelength range belonging to wavelengths from 1050 nanometers to 1350 nanometers, the amount of the transmitted light being measured for each pixel, using an area sensor having a pixel area of h×w for the amount of the transmitted light; and determining that a blood coagulation reaction is finished or not finished, depending on a value of correlation coefficient r. As a result, completion of the blood coagulation reaction can be determined by a new viewpoint. This is takes advantage of a characteristic of the amount of the transmitted light being proportional to the optical path length, and uses, as a basis for determination, the correlation coefficient between the intensity of the transmitted light at a specific pixel of the area sensor that is the light receiving element and the optical path length in the vessel. The time at which a state deviating from the proportional relationship occurs can be set as the completion of the blood coagulation reaction.

The method of determining blood coagulation reaction completion according to claim 10 is characterized in that when the correlation coefficient is 0.8 or less, the blood coagulation reaction is determined to be finished. In this manner, by setting the correlation coefficient of 0.8 as a reference, the accuracy of the determination of completion of blood coagulation reaction can be increased.

An automated blood centrifuge device according to claim 11 is characterized in having: a centrifugal separating mechanism used in a laboratory test for performing centrifugal separation for a blood collection tube containing a measurement sample obtained by mixing whole blood and a blood coagulation accelerator; a measurement light source capable of irradiating the blood collection tube with a near infrared light of a specified wavelength range belonging to a range from 1050 nanometers to 1350 nanometers; a light intensity measuring mechanism that measures an amount of light of the specified wavelength range transmitted through an inside of a specimen in the blood collection tube; a coagulation reaction determining mechanism that determines that a coagulation reaction is in progress when the amount of the light exhibits an increasing trend and that determines that the coagulation reaction is finished when the amount of the light exhibits a decreasing trend; and a conveying mechanism that conveys, to the centrifugal separating mechanism, the blood collection tube for which the coagulation reaction is determined to be finished. As a result, it becomes possible to subject only a blood collection tube for which the coagulation reaction is finished to centrifugation treatment.

The automated blood centrifuge device according to claim 12 is characterized in that, as a particularly favorable wavelength range, a wavelength of the specified wavelength range is present in a range from 1250 nanometers to 1300 nanometers.

The automated blood centrifuge device according to claim 13 characterized in that, more favorably, a peak wavelength of the measurement light source is a LED light source of a single wavelength in a range from 1050 nanometers to 1350 nanometers.

The automated blood centrifuge device according to claim 14 is characterized in further having: a specimen buffer mechanism that temporarily holds the blood collection tube; and a transporting mechanism that transports, to the specimen buffer mechanism, the blood collection tube for which the coagulation reaction is determined to be in progress by the coagulation reaction determining mechanism, and after waiting for a predetermined time, again, returns the blood collection tube to the light intensity measuring mechanism for measurement of an intensity of the light. As a result, the blood collection tube for which the coagulation reaction is determined to be in progress is held by the specimen buffer mechanism for a predetermined time, whereby the intensity of the light transmitted through the inside of the specimen in the blood collection tube for which the coagulation reaction was determined to be in progress can be measured accurately.

An automated blood centrifuge device according to claim 15 is characterized in having: a centrifugal separating mechanism for a laboratory test; a mechanism that irradiates a blood collection tube with a near infrared light having a peak wavelength in a range from 1050 nanometers to 1350 nanometers; a mechanism that measures, using an area sensor that has a pixel region of h×w, temporal variation of an intensity of a transmitted light of the near infrared light irradiated to the blood collection tube; and a coagulation reaction determining mechanism that determines whether a blood coagulation reaction has finished, based on an amount of change of the intensity of the transmitted light per unit time. The coagulation reaction determining mechanism determines that the blood coagulation reaction has finished or has not finished, depending on a value of a correlation coefficient r, and has a conveying mechanism that conveys, to the centrifugal separating mechanism, the blood collection tube for which the blood coagulation reaction is determined to be finished. As a result, completion of the blood coagulation reaction can be determined by a new viewpoint. This is takes advantage of a characteristic of the amount of the transmitted light being proportional to the optical path length, and uses, as a basis for determination, the correlation coefficient between the intensity of the transmitted light at a specific pixel of the area sensor that is the light receiving element and the optical path length in the vessel. The time at which a state deviating from the proportional relationship occurs can be set as the completion of the blood coagulation reaction.

The automated blood centrifuge device according to claim 16 is characterized in that when the correlation coefficient is 0.8 or less, the blood coagulation reaction is determined to be finished. In this manner, by setting the correlation coefficient of 0.8 as a reference, the accuracy of the determination of completion of blood coagulation reaction can be increased.

EFFECT OF THE INVENTION

The present invention enables detection of the blood coagulation reaction process for whole blood in a blood collection tube, measuring tube, etc. Thus, detection of the blood coagulation reaction is easier and simpler than conventionally. Further, the process of a clinical laboratory technologist visually determining the completion of the blood coagulation reaction is automated, thereby enabling preprocessing work for a centrifuge device to be automated and enabling enhancement of the efficiency of laboratory tests.

BRIEF DESCRIPTION OF DRAWINGS

Fig. 1 is a diagram depicting an example of a configuration of a blood coagulation measuring device according to a first embodiment.
Fig. 2 is a diagram depicting a blood collection tube surface and an imaging area of an infrared camera used in the first embodiment.

Fig. 3 is a graph depicting intensity of transmitted light and wavelength characteristics of near infrared light according to the first embodiment.

Fig. 4 is a graph depicting transmitted light intensity curves of multiple test subjects measured over time by the blood coagulation measuring device according to the first embodiment.

Fig. 5 is a graph depicting a transmitted light intensity curve when the transmitted light intensity of a single specimen according to a second embodiment is measured for a long period.

Fig. 6 is a graph depicting temporal variation of a correlative relationship between optical path length in a blood collection tube and the transmitted light intensity of each measurement pixel of an imaging area in a transitional process of the blood coagulation reaction according to the second embodiment.

Fig. 7 is a schematic diagram of a measuring tube suitable for the blood coagulation measuring device according to a third embodiment.

Fig. 8 a schematic diagram depicting the blood coagulation measuring device according to the third embodiment.

Fig. 9 is a diagram depicting an example of configuration of the blood coagulation measuring device according to a fourth embodiment.

Fig. 10 is a graph depicting results of measuring transmitted light and scattered light concurrently according to the fourth embodiment.

Fig. 11 is a diagram depicting an example of a configuration of the automated blood centrifuge device according to a fifth embodiment.

Fig. 12 is a flowchart depicting processes of the automated blood centrifuge device according to the fifth embodiment.

Fig. 13 is a graph depicting results of concurrent measurement of transmitted light (solid line) and scattered light (dashed line) according to a sixth embodiment, over a long period.

BEST MODE (S) FOR CARRYING OUT THE INVENTION

Herein, while embodiments of the present invention are described, the present invention is not limited to the embodiments.

(First embodiment)

(Description of blood collection tube and measuring system)

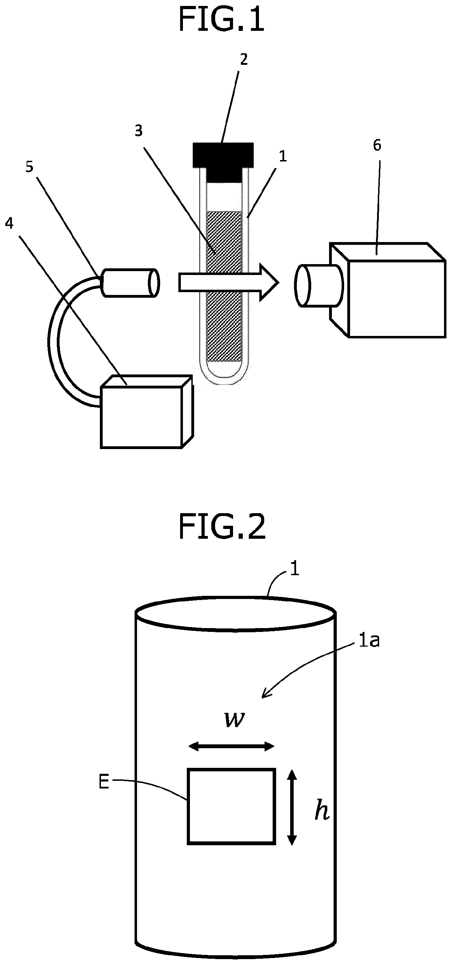

Fig. 1 is a diagram depicting an example of a configuration of a blood coagulation measuring device according to a first embodiment. First, a blood specimen and blood collection tube for measurement related to the present invention are described. In the actual analysis of a specimen at a medical facility, various types of blood collection tubes are selectively used according to purpose. The blood collection tubes may contain a pre-added anticoagulant agent, blood coagulation accelerator, separating agent that improves separation of serum and a blood clot or separation of blood plasma and hemocyte, etc. For example, in an instance of a specimen for which blood coagulation is to be measured, a blood specimen is collected in a blood collection tube that contain an anticoagulant agent such as a citrate. Further, typically, in an instance of a blood centrifugation treatment for separating the serum, a blood specimen is collected in a blood collection tube to which a blood coagulation accelerator such as silica particles or thrombin has been added.

Nonetheless, when a blood specimen is collected in a blood collection tube that contains a blood coagulation accelerator, the blood coagulation reaction starts immediately and monitoring of near infrared light from the starting point of the reaction is difficult. Thus, in a verification experiment of the present invention, for convenience, even in the experiment, which assumed a blood centrifugation treatment for serum separation, first, the blood specimen was collected in a blood collection tube that contained an anticoagulant agent and monitoring of near infrared light was performed and after these preparations, a blood coagulation accelerator was added, whereby the starting point of the reaction could be clearly known. In this verification experiment, "Activated Partial Thromboplastin Time Kit Thrombo Check APTT" (Sysmex Corporation) was used as the blood coagulation accelerator. This reagent is composed of an APTT reagent that contains cephalins and an ellagic acid derived from rabbit brains, and two types of solutions of 0.02M calcium chloride solution. The start of the blood coagulation reaction was assumed to be the point of mixing by gentle inversion at least five times after the addition of both liquids. Due to the inversion mixing, the blood coagulation reaction due to fibrin formation proceeds uniformly, whereby the starting point of the reaction can be identified more accurately. In the present invention, in the description hereinafter, this state is referred to as "specimen".

Next, the blood coagulation measuring device is described. A halogen light source (BlueVision, Co., Ltd., Model: BV-M1021) with a variable wavelength from 900 nanometers to 1800 nanometers was used as a near infrared light source (measurement light source) 4. Irradiated light of the wavelength selected via a dial was led out by an optical fiber 5; an emitting surface was near a blood collection tube 1 containing whole blood 3; and the blood collection tube 1 was irradiated with near infrared light. Reference numeral 2 is a cap, and an irradiation area of the blood collection tube 1 had a round shape with a diameter of about 20mm. As an infrared camera 6 constituting a measuring unit, a short-wavelength infrared camera (BlueVision, Co., Ltd., Model: BV-C2900) was used as an area sensor, and a frame frequency of measurement images was 20 hertz or 10 hertz per second.

Fig. 2 is a diagram depicting the blood collection tube surface and an imaging area of the infrared camera used in the first embodiment. An imaging area E of the blood collection tube surface 1a, specifically, was assumed as a region 50 pixels in height (h) × 50 pixels in width (w) (a 7mm×7mm square in a cross-section of the blood collection tube 1), in a center portion of the blood collection tube 1, the center portion being relative to the length of the blood collection tube 1.

Calculation of the intensity of the transmitted light was obtained, by a computer, as an average of the entire imaging area E (50 pixels×50 pixels), by inputting into the computer, luminance values of the pixels of the imaging area E (50 pixelsx50 pixels) output by the infrared camera 6.

(Wavelength characteristics of transmitted light)

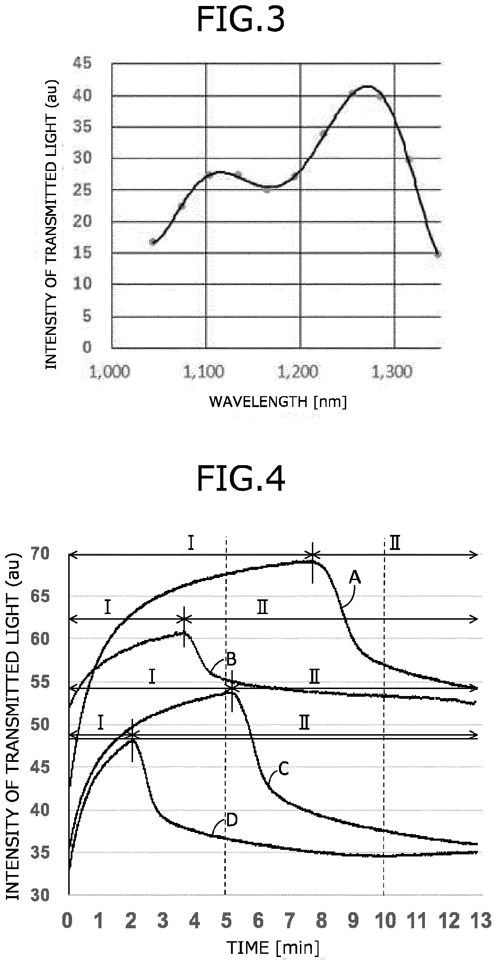

Fig. 3 is a graph depicting the intensity of the transmitted light and wavelength characteristics of the near infrared light according to the first embodiment. A horizontal axis indicates wavelength [nanometers: nm] while a vertical axis indicates transmitted light intensity (au). Fig. 3 depicts results of investigating the maximum absorption wavelength in the near infrared region by using a specimen in which the blood coagulation reaction was in progress. As depicted in Fig. 3, regarding transmission characteristics of the near infrared light with respect to the specimen, it is found that transmittance for a range from 1050 nanometers to 1350 nanometers is particularly high, and in the blood coagulation measurement of the present invention, particularly around 1270 nanometers, the intensity of the transmitted light is the highest and suitable. In a wavelength region of 1050 nanometers or less, due to the effects of scattering and absorption due to the hemocyte, and in a region of 1350 nanometers or more, water component absorption was great and the transmitted light was small.

As a result, in the measurement below, a wavelength of 1270 nanometers was selected. Furthermore, stable transmission of near infrared light of the 1270-nanometer wavelength is possible even when a label (limited to a label free of a substance that blocks or absorbs near infrared light such as a metal or a specific inorganic substance) is affixed to the blood collection tube 1. In particular, in the measurement, it is more suitable when the label is affixed to the image sensor side. As a result, it was found to be more convenient in practice as compared to visual inspection using visible light.

(Determination of coagulation reaction completion)

Fig. 4 is a graph depicting transmitted light intensity curves of multiple test subjects measured over time by the blood coagulation measuring device according to the first embodiment. A horizontal axis indicates time [min], a vertical axis indicates transmitted light intensity (au). Fig. 4 depicts results when the transmitted light intensity was measured over time using near infrared light with a wavelength of 1270 nanometers and, as the starting point, a point when a suitable amount of a blood coagulation accelerator was added to the whole blood of four test subjects (A to D). The test subjects A to D show that peak times and transmitted light intensities differ.

A visual state in the blood collection tube 1 is initially a uniform, a fresh blood color that eventually changes to a darker red color and eventually changes into a gel or agar like form as involution progresses. The change in the transmitted light intensity was common to all four test subjects and was found to include a first process I (referred to as a coagulation activation process) of rapid increase with the elapse of time immediately after the inversion mixing and a second process II (referred to as a clot involution process) of rapid decrease with a peak as a boundary.

In the first process I, from an initial state in which the hemocyte, such as red blood cells, is uniformly free and suspended, a fine aggregated structure of the hemocyte is formed accompanying fibrin formation, gap regions between a blood clot and the serum appear and therefore, it can be surmised that the transmitted light intensity increases. Further, in the second process II, a dense structure is formed due to blood clot formation, whereby transmitted light is blocked and thus, it can be surmised that the transmitted light intensity decreases.

Utilizing these results, blood coagulation time can be measured by measuring from the time when the blood coagulation accelerator is mixed until the transmitted light intensity switches from a state of increasing to a state of decreasing, that is, the time indicating the maximum value. Further, a new blood coagulation measuring device can also be manufactured.

In this manner, the inventors found that the process of the blood coagulation reaction can be observed, by using the near infrared light source 4 to irradiate the blood collection tube 1 containing the whole blood 3 with near infrared light of a specified wavelength in a range of 1050 nanometers to 1350 nanometers and measuring temporal variation of the intensity of the light that is transmitted through the whole blood 3 and detected by the infrared camera 6.

(Determination of completion of blood coagulation reaction)

In Fig. 4, when looking at the variation of the transmitted light intensity at an arbitrary time after the inversion mixing of the whole blood and the blood coagulation accelerator, for example, at a time point 5 minutes thereafter, it can be read that depending on the specimen, an increasing trend is exhibited or a decreasing trend is exhibited. In this instance, for a specimen (for example, test subjects A, C) exhibiting an increasing trend, the coagulation reaction can be determined to be in progress whereas for a specimen exhibiting a decreasing trend (for example, test subjects B, D), the coagulation reaction can be determined to be completed.

Thus, by measuring variation of the transmitted light intensity at a predetermined time after the inversion mixing, a selection process becomes possible in which a specimen exhibiting a decreasing trend is placed in a centrifuge, and a specimen exhibiting an increasing trend is sent to a specimen buffer mechanism and after the elapse of the subsequent predetermined time, the same measurement is performed. By these procedures, the conventional determination of coagulation reaction completion performed visually by a clinical laboratory technologist can be performed automatically.

As a result, it becomes possible to perform centrifugation treatment only on specimens for which the coagulation reaction has been completed with certainty, and serum free of remaining fibrinogen and/or fibrin can be easily obtained.

(Second embodiment)

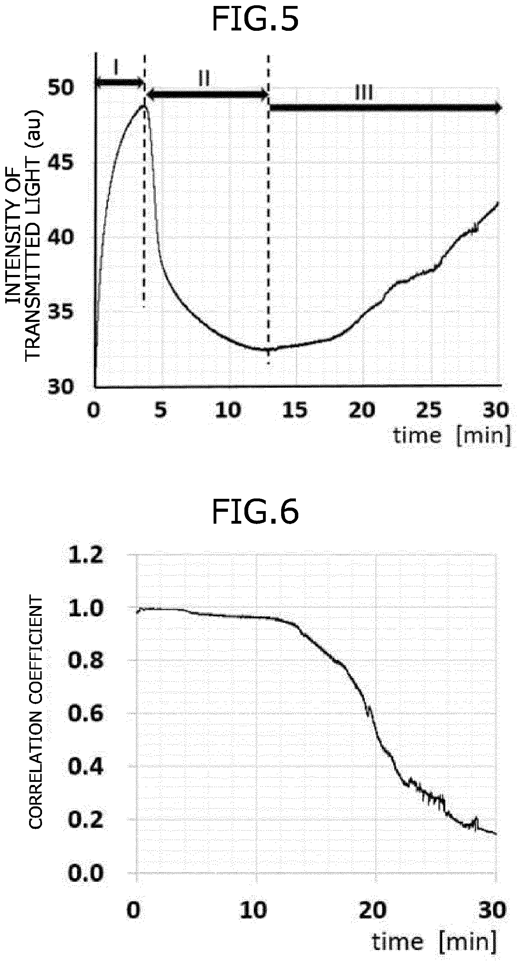

Fig. 5 is a graph depicting a transmitted light intensity curve when the transmitted light intensity of a single specimen according to a second embodiment is measured for a long period. Fig. 5 depicts results of monitoring one of the specimens in Fig. 4 for a longer time, for example, 30 minutes. Further, as depicted in Fig. 5, the transmitted light intensity exhibits an increasing trend from a bottom of the curve around the 13-minute mark. Here, in a third process III after the first process I and the second process II described by Fig. 4, the transmitted light intensity increases after passing a minimum point. This can be interpreted to be a state in which after completion of the blood coagulation reaction, serum penetrates a more contracted region accompanying higher order structural changes due to involution of blood clots.

The blood coagulation reaction has already finished and therefore, the third process III indicates that there is a risk of the coagulation reaction being determined to be incomplete in the method of determining completion of the coagulation reaction by a decreasing trend of the transmitted light intensity after the elapse of the predetermined time described above (for example, after the elapse of 5 minutes as described by Fig. 4).

In the blood coagulation reaction process, the inventor verified that in the first process I and the second process II, absorption behavior of near infrared light due to the hemocyte, etc. in the blood collection tube 1 follows the Lambert-Beer law and is directly proportional to the optical path length passing through the blood collection tube 1.

Here, a common logarithm of a transmitted light intensity $\mu_i$ at a pixel position i the infrared camera 6 (area sensor) is expressed by $\omega_i$ (equation (1) below). A correlation coefficient r here is expressed by equation (2) below.

$$\omega_i = \log_{10} \mu_i \quad \dots (1)$$

$$r = \frac{\sum_{i=1}^{n}\left(l_i - \bar{l}\right)(\omega_i - \bar{\omega})}{\sqrt{\sum_{i=1}^{n}\left(l_i - \bar{l}\right)^2 \sum_{i=1}^{n}(\omega_i - \bar{\omega})^2}} \quad \dots (2)$$

Where, $l_i$ represents the optical path length in the blood collection tube 1 at the measurement pixel position i. In an instance of the present measurement, the optical path length is equal to an inner diameter of the center portion of the blood collection tube 1 and zero at both transverse faces.

Fig. 6 is a graph depicting temporal variation of a correlative relationship between the optical path length in the blood collection tube and the transmitted light intensity of each measurement pixel of an imaging area in a transitional process of the blood coagulation reaction according to the second embodiment. A horizontal axis indicates time [min] and a vertical axis indicates correlation coefficients. Fig. 6 indicates that an initial correlation coefficient hovers around 1 but eventually begins to fall.

In comparing Fig. 5 and Fig. 6, until around the 13-minute mark, the correlation coefficient is substantially close to 1 and while variation of the transmitted light intensity in the first process I and the second process II in Fig. 5 indicates high correlation with the optical path length, after the 13-minute mark, the correlation coefficient markedly decreases and is less than 0.8. This can be interpreted to mean that involution of the blood clot after completion of the blood coagulation reaction further progresses, whereby the shape of blood clot in the imaging area becomes less uniform and thus, the correlation between the optical path length and the transmitted light intensity at the pixel position i of the short-wavelength infrared camera 6 is small. As a result, even when the transmitted light intensity exhibits an increasing trend, when the correlation coefficient is 0.8 or less, the blood coagulation reaction needs to be determined as having already been completed.

In this manner, the infrared camera 6 (area sensor) is used as the light receiving element and the correlation coefficient between the transmitted light intensity and the optical path length is investigated, whereby it becomes possible to determine that the blood coagulation reaction has finished. Further, the method described in the second embodiment can be combined with the method described in the first embodiment to further enhance the reliability of the results of the determination.

(Third embodiment)

(Blood coagulation measuring device)

The present invention is a method of directly measuring, by red blood cells, which comprise a large part of the hemocyte component, absorption characteristics of near infrared light of a specified wavelength range belonging to 1050 nanometers to 1350 nanometers, and monitoring coagulation process. Thus, use as a dedicated blood coagulation measuring device is possible by using a measuring tube suitable for a small amount of a whole blood specimen, instead of the blood collection tube 1 described above.

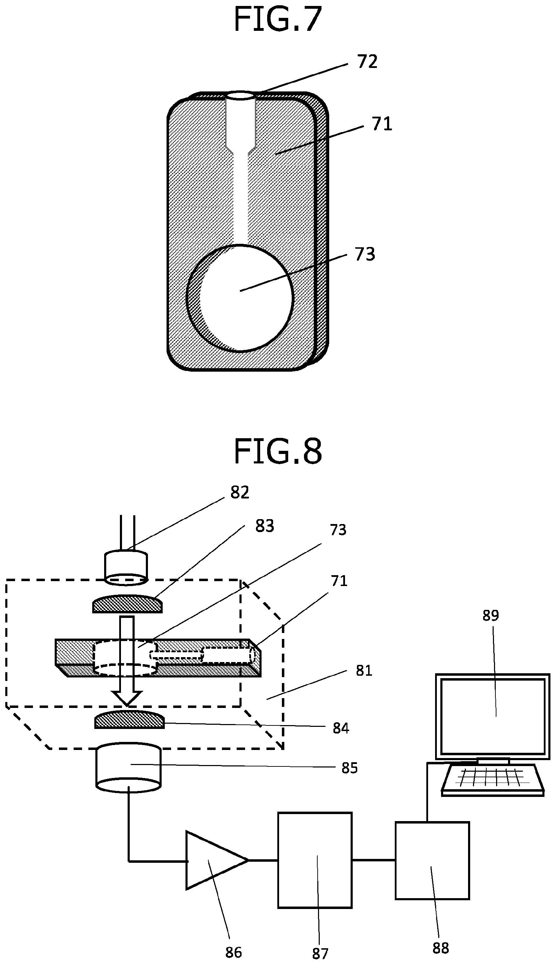

Fig. 7 is a schematic diagram of a measuring tube suitable for the blood coagulation measuring device according to the third embodiment. Herein, an example of a measuring tube 71 is described. For example, the measuring tube 71 is a vessel containing a polypropylene. The measuring tube 71 has an inlet 72 for injecting a specimen into a measuring section 73.

Specifically, for example, the measuring section 73 has a hollow section with a diameter of 8 millimeters, a thickness of 5 millimeters, and a capacity of 0.25 milliliters, and there is no absorption of near infrared light in the measuring section 73. In advance, 10 microliters of the APTT reagent are injected through the inlet 72 by pipet, in addition to this, 0.2 milliliters of the specimen described in the first embodiment is added to the measuring section 73, and a gentle mixing treatment is performed.

Fig. 8 a schematic diagram depicting the blood coagulation measuring device according to the third embodiment. The blood coagulation measuring device depicted in Fig. 8 has a configuration in which the measuring tube 71 is inserted between a near infrared light source 82 that is compact and emits near infrared light and a light receiving element 85 sensitive to near infrared light. The measuring tube 71 that contains the specimen is set horizontally in a thermostatic device 81 adjusted to, for example, 37 degrees C. As a result, the effects of gravity when the hemocyte settles can be reduced.

To reduce the size of the measuring device, one example, a commercial LED light source such as that of Hamamatsu Photonics K.K., Model: L12771 (peak wavelength: 1300 nanometers) is used as the near infrared light source 82 and is disposed above the specimen. Further, a InGaAsPIN-type photodiode is suitable as the light receiving element 85, and that of Hamamatsu Photonics K.K., Model: G12180-02A or the like is disposed beneath the measuring tube 71.

Light emitted from the near infrared light source 82 is collimated by a convex lens 83 and irradiated on the measuring section 73. Light transmitted by the measuring section 73 is collected to a light receiving area of the light receiving element 85 by a collecting lens 84, and information about the intensity of the transmitted light can be obtained. A light receiving circuit unit is configured by an amplifier circuit 86, an A/D converting circuit 87, and a memory unit 88, and the light receiving circuit unit amplifies, digitally converts, and then accumulates a photocurrent generated by a light receiving element. Information regarding the accumulated transmitted light intensity is processed by an external arithmetic processing device 89 and the amount of time that elapses until a maximum value of the transmitted light intensity is indicated is measured, whereby the blood coagulation time can be obtained. By such a configuration, a compact blood coagulation measuring device can be obtained.

(Fourth embodiment)

In a fourth embodiment, a technique of using scattered light is described with respect to the technique of using the transmitted light described above. The wavelength of the near infrared light used in the present invention is in a range of 1050 nanometers to 1350 nanometers belonging to a short-wavelength infrared region and in contrast, the size of the red blood cells comprising a large part of the hemocyte has a diameter of 7 to 8 micrometers. Therefore, Rayleigh scattering occurs similarly as with the visible light used in the conventional blood coagulation measurement.

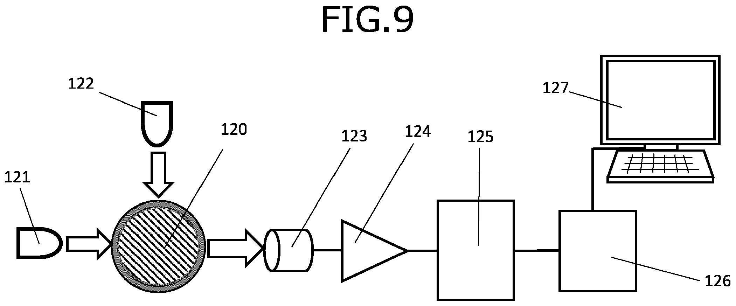

Fig. 9 is a diagram depicting an example of configuration of the blood coagulation measuring device according to the fourth embodiment. A LED 121 and a LED 122 (both being Model: L12771-1, Hamamatsu Photonics K.K.) are used and are disposed orthogonal to and close to a center of the blood collection tube 120 containing whole blood and a blood coagulation accelerator that have been mixed by inversion mixing. Here, near infrared light irradiated from the LEDs is about 4mm in diameter and incident on the specimen with the luminous flux being concentrated by lenses of all surfaces. As the light receiving element, a light receiving element 123 (Model: G12180-02A, Hamamatsu Photonics K.K.) of a single pixel formed by InGaAs is disposed in a linear direction of the LED 121 and so that the two LEDs and the light receiving element are coplanar in a periphery of the blood collection tube 120. As a result, transmitted light from the LED 121, and right-angled scattered light from the LED 122 are incident to the light receiving element 123.

Configuration may be such that the LED 121 and the LED 122 are driven independently of each other, and the transmitted light alone or the scattered light alone is measured. Further, in Fig. 9, while the LED 122 and the light receiving element 123 are disposed orthogonally, an angle other than this can be selected. Furthermore, by alternating the emission times of the two LEDs 121 and 122, mutual interference can be eliminated, whereby the scattered light and the transmitted light can be measured concurrently. As a result, the light receiving circuit can be configured by a single system, whereby the measuring device can be configured at a lower cost as compared to a configuration constituted by a light receiving circuit having a single LED and two systems.

Next, the measuring unit is described. Photocurrent generated by the light receiving element 123 is digitized by an amplifying unit 124 constituted by an operational amplifier or the like similarly to a conventional technique and an AID converting unit 125 that converts analog information into digital information. Subsequently, the digital information is stored to a memory unit 126, and the time that elapses until the amount of transmitted and/or scattered light changes from increasing to decreasing is measured by a control unit 127.

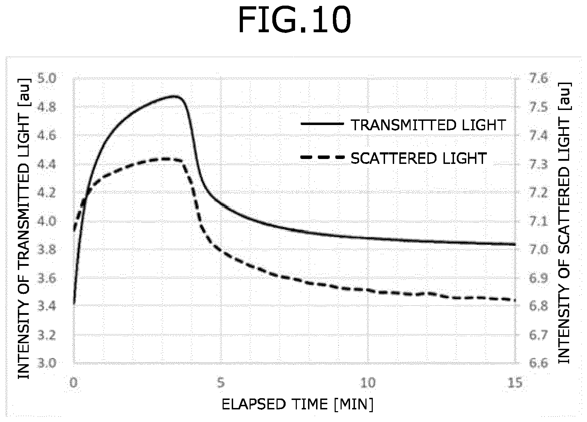

Fig. 10 is a graph depicting results of measuring transmitted light and scattered light concurrently according to the fourth embodiment. In the present embodiment, the intensity of scattered light indicated by the dashed line is greater as compared to the transmitted light indicated by a solid line and therefore, during this measurement, more current was input to the LED 121 than the LED 122 to increase the light emission luminance. As a result, regarding the first process I and the second process II of the blood coagulation process, it is found that similarly to that depicted in Fig. 4, around the 4-minute mark, a changing point from an increasing trend to decreasing trend is observed and the trends for the transmitted light and the scattered light are substantially the same.

From the results in Fig. 10, it is found that, for the blood coagulation measuring device, in the measurement by the near infrared light of the present invention, the permeability of the light is high and therefore, the transmitted light alone or the scattered light alone can be measured. In the conventional blood coagulation measurement using visible light, scattering occurs near the surface of the specimen and therefore, measurement by the transmitted light is difficult whereas with the near infrared light of the present invention, measurement by the transmitted light is possible, and there is a further advantage in that even in an instance of scattered light, information about the structure occurring deep in the specimen can be obtained.

(Fifth embodiment)

In a fifth embodiment, an automated blood centrifuge device that uses the above method is described. The automated blood centrifuge device implements preprocessing mainly up to insertion of a collected blood specimen into a centrifugal separating mechanism. The automated blood centrifuge device may be configured to include the centrifugal separating mechanism.

Similarly as described above, a blood specimen of 7 milliliters is collected in a blood collection tube containing a 3.2% sodium citrate solution (anticoagulant agent) and a separating agent. As a blood coagulation accelerator, two types of reagents of the Activated Partial Thromboplastin Time Kit Thrombo Check APTT (Sysmex Corporation) described above are added for every 200 microliters, and sealing and inversion mixing are performed whereby the measurement sample is prepared.

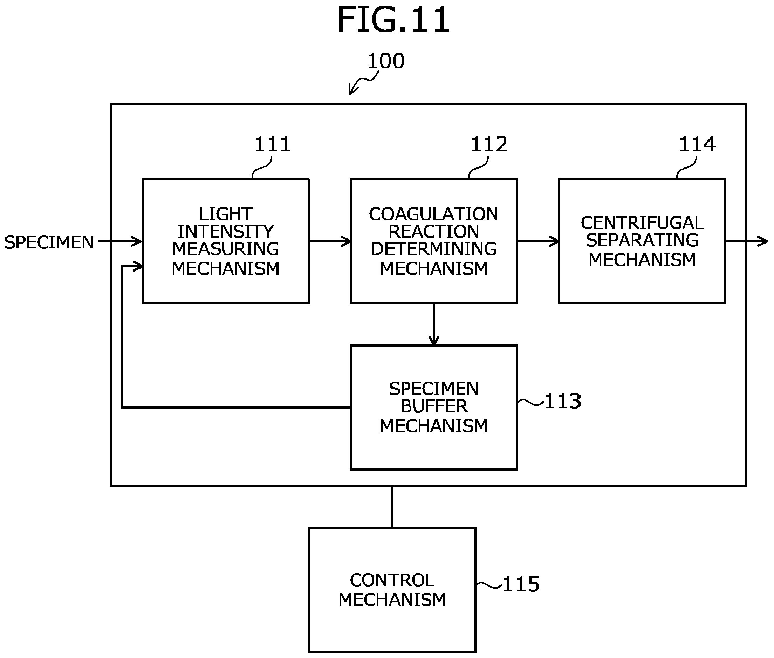

Fig. 11 is a diagram depicting an example of a configuration of the automated blood centrifuge device according to the fifth embodiment. An automated blood centrifuge device 100, via mechanisms, automatically and without human intervention, conveys the specimen containing whole blood including the blood coagulation accelerator to the centrifugal separating mechanism. The automated blood centrifuge device 100 has a light intensity measuring mechanism 111, a coagulation reaction determining mechanism 112, a specimen buffer mechanism 113, a centrifugal separating mechanism 114, a specimen conveying mechanism (not depicted), and a control mechanism 115.

The light intensity measuring mechanism 111 measures variation of the light intensity of the measurement sample set in the automated blood centrifuge device 100, for a predetermined amount of time (for example, 5 seconds as described above). The coagulation reaction determining mechanism 112 determines completion/incompletion of the blood coagulation reaction, based on the trend of the variation of the light intensity.

When the blood coagulation reaction is determined to be incomplete, the specimen is temporarily conveyed to the specimen buffer mechanism 113 and is held for a predetermined time (for example, 7 minutes). Thereafter, the specimen is again returned to the light intensity measuring mechanism 111 and the coagulation reaction determining mechanism 112, and the completion/incompletion of the blood coagulation reaction is determined.

The coagulation reaction determining mechanism 112 conveys the specimen for which the blood coagulation reaction has been determined to be completed to the subsequent centrifugal separating mechanism 114, and the centrifugal separating mechanism 114 performs a centrifugation treatment on the specimen. On the other hand, when the coagulation reaction determining mechanism 112 determines that the blood coagulation reaction is incomplete, the specimen is again returned to the specimen buffer mechanism 113. The specimen buffer mechanism 113 discharges from the automated blood centrifuge device 100 as an abnormal specimen, the specimen for which the blood coagulation reaction has been determined to be incomplete despite being reprocessed.

The control mechanism 115 is constituted by the mechanisms of the automated blood centrifuge device 100, and comprehensively controls each control operation of the light intensity measuring mechanism 111, the coagulation reaction determining mechanism 112, the specimen buffer mechanism 113, the centrifugal separating mechanism 114, and the specimen conveying mechanism (not depicted). Here, the mechanisms of the automated blood centrifuge device 100 may have independent control mechanisms, respectively, or control operations of the mechanisms can be centralized in a single unit of the control mechanism 115.

Here, the control mechanism 115 can implement processing for determining of completion of blood coagulation reaction by the coagulation reaction determining mechanism 112. For example, as described in the first embodiment above, each component (the near infrared light source 4, the infrared camera 6) for the blood coagulation measurement is controlled.

Further, the control mechanism 115 controls the conveyance of a specimen exhibiting a decreasing trend, to the centrifugal separating mechanism 114. On the other hand, for a specimen exhibiting an increasing trend, conveyance to the specimen buffer mechanism 113 is controlled and a selection process of performing the same measurement after the subsequent predetermined time elapses is performed. By these procedures, it becomes possible to automate the conventional determination of coagulation reaction completion performed visually by a clinical laboratory technologist.

Further, as described in the second embodiment above, the infrared camera 6 (area sensor) is used in the blood coagulation measurement and the control mechanism 115 (the coagulation reaction determining mechanism 112) executes processing by an algorithm that uses correlation coefficients, whereby the accuracy of the determination of the blood coagulation measurement can be further increased.

The control mechanism 115 of the automated blood centrifuge device 100 can be configured using a hardware configuration similar to a general computer device having a CPU, ROM, RAM, etc.

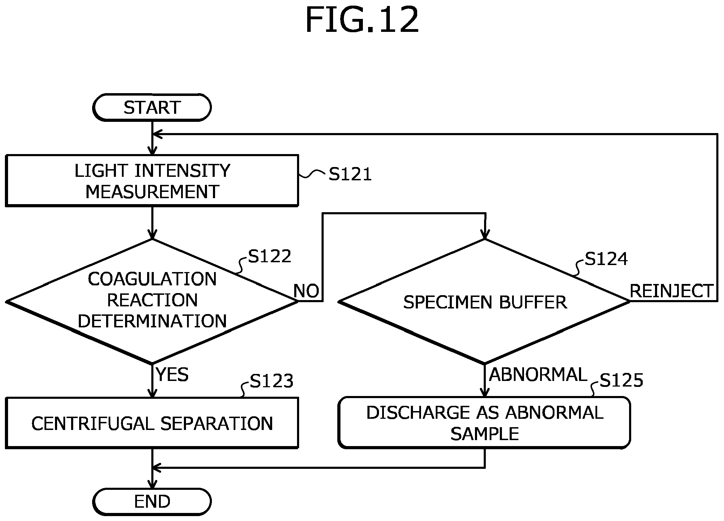

Fig. 12 is a flowchart depicting processes of the automated blood centrifuge device according to the fifth embodiment. Fig. 12 mainly depicts the contents of the processes implemented by the control mechanism 115 described above. When a specimen in which collected blood and the blood coagulation accelerator have been treated by the inversion mixing is set in the automated blood centrifuge device 100, the control mechanism 115 conveys the specimen to the light intensity measuring mechanism by the conveying mechanism.

At this time, in the specimen, the coagulation reaction is already in progress and the elapsed time is uncertain. Here, the control mechanism 115 causes the light intensity measuring mechanism 111 to measure changes in the light intensity for five seconds (step S121). While the measurement time is affected by noise on the measuring side and the intensity of the source light which is near infrared light, normally, a few seconds to about 10 seconds is sufficient.

Thereafter, the control mechanism 115 causes the coagulation reaction determining mechanism 112 to detect changes in the light intensity of the specimen and to judge the coagulation reaction of the blood (step S122). Here, in an instance in which the light intensity of the specimen exhibits an increasing trend, the control mechanism 115 determines that the blood coagulation reaction is not finished (step S122: NO), and transitions to the process at step S124. On the other hand, in an instance in which the light intensity of the specimen exhibits a decreasing trend, the control mechanism 115 determines that the blood coagulation reaction has finished (step S122: YES), and transitions to the process at step S123.

At step S123, according to the determination, the control mechanism 115 causes a specimen for which the blood coagulation reaction has been determined to be completed to be conveyed to the subsequent centrifugal separating mechanism 114 by the conveying mechanism and terminates the processes above. The centrifugal separating mechanism 114 performs centrifugation treatment on the specimen.

Meanwhile, at step S124, the control mechanism 115 causes a specimen for which the blood coagulation reaction has been determined to be not finished to be conveyed temporarily to the specimen buffer mechanism 113 by the conveying mechanism. The control mechanism 115, after the specimen conveyed to the specimen buffer mechanism 113 has been held for a predetermined time (for example, 7 minutes), again, reinjects the specimen into the process at step S121 (the light intensity measuring mechanism 111) (step S124: reinject), and implements the processes at step S121 and step S122 (the coagulation reaction determining mechanism 112). On the other hand, after conveyance of a specimen to the specimen buffer mechanism 113, the control mechanism 115 causes a specimen (step S124: abnormal) for which the blood coagulation reaction is determined to not be finished at step S122 (step S122: NO) to be discharged from the automated blood centrifuge device 100 as an abnormal specimen (step S125) and terminates the series of processes. The discharged abnormal specimen is processed separated.

(Sixth embodiment)

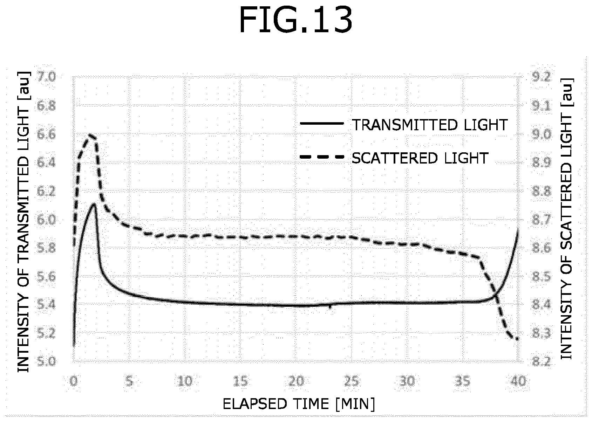

In a sixth embodiment, a working example is described in which the present invention is used in the automated blood centrifuge device depicted in Fig. 9 above. Fig. 13 is a graph depicting results of concurrent measurement of transmitted light (solid line) and scattered light (dashed line) according to the sixth embodiment, over a long period. Here, "concurrent" means that two or more beams of light are irradiated on a single specimen from different directions within a specified unit time and as a result, different types of light are emitted within the unit time. While the first process I and the second process II of the blood coagulation process exhibit substantially the same trends as in the example depicted in Fig. 10, in the third process III, the behavior of the transmitted light and of the scattered light differ greatly and contrary to the increase in the amount of transmitted light, the amount of scattered light decreases.

Regarding changes in the amount of transmitted light of the third process III, it is presumed that loses in light are due to a fibrinolytic reaction due to fibrin thrombolysis, etc. in blood clots. On the other hand, regarding changes in the amount of scattered light of the third process III, it is presumed that due to involution of the blood clot, serum that is gel-like and contains water in a periphery as a main component occurs, whereby a difference in the refractive index occurs and in particular, reflected light increases.

As a result, in the third process III, differences in the amount of the transmitted light and the amount of the scattered light that are seen can be used as described below. In particular, in an instance in which the determination of the completion of the blood coagulation reaction relies on information about increases in the amount of light during an arbitrary short time, it is difficult to determine whether the state is the first process I or the third process III using only the information about the amount of transmitted light. However, as depicted in Fig. 13, even in an instance in which the amount of the transmitted light is increasing and the amount of the scattered light is decreasing, as the third process III, the coagulation reaction can be determined to have finished. In this manner, information about the amount of transmitted light and the amount of scattered light may be measured concurrently, whereby the determination of the first process I and the third process III can be made more reliable.

According to the processes above, it becomes possible to put, in the centrifugal separating mechanism 114, only a specimen for which the blood coagulation reaction is finished.

As described above, according to the present invention, the blood coagulation reaction can be easily detected and therefore, use for a new blood coagulation measuring device is possible. Further, the blood coagulation reaction process can be detected while the whole blood is in a blood collection tube or measuring tube and therefore, it becomes possible to put only specimens for which the blood coagulation reaction has finished into the centrifugal separating mechanism thereafter. As a result, after centrifugation, a serum layer free of fibrinogen and fibrin can be obtained and as a result, the problem of the dispensing nozzle clogging when the serum is dispensed can be prevented in advance. Furthermore, determination of completion of blood coagulation reaction, which has been performed visually can be automated and therefore, preprocessing work for a centrifuge device can be automated, whereby laboratory tests can be made labor-saving and more efficient.

Further, even in an instance in which a paper label is affixed to the infrared camera side of the blood collection tube, near infrared light is favorably transmitted and therefore, one advantage of the present invention is that blood coagulation measurement can be performed reliably, without optical hinderances.

The present invention is a technology for easily detecting processes of a blood coagulation reaction by arithmetically processing changes in the amount of short-wavelength infrared light passing through the inside of a specimen. Changes in the amount of light are based on, as described above, a fine aggregated structure of hemocyte being formed accompanying fibrin formation from the initial state in which the hemocyte is uniformly free and suspended, gap regions between a blood clot and the serum appearing, a dense structure being formed due to blood clot formation, etc. and it has little effect on which mechanism of action the resulting blood clotting reaction is due to on the coagulation cascade. Therefore, the present invention can be widely applied to an application (for example, a blood coagulation measuring device) such as collecting a blood specimen by a blood collection vessel containing an anticoagulant agent and an application (for example, a coagulation completion determination method before blood centrifugation treatment for the purpose of serum separation) such as collecting a blood specimen using a blood collection tube that contains blood coagulation accelerator, and may be said to be extremely useful.

INDUSTRIAL APPLICABILITY

The present invention can be applied to technology related to blood coagulation testing and in particular, is useful for automating preprocessing work of centrifugation treatment of whole blood.

EXPLANATIONS OF LETTERS OR NUMERALS

1 blood collection tube
2 cap
3 whole blood
4, 82 near infrared light source (measurement light source)
5 optical fiber
6 infrared camera
71 measuring tube
72 inlet
73 measuring unit
81 thermostatic device
83 convex lens
84 collecting lens
85 light receiving element
100 automated blood centrifuge device
111 light intensity measuring mechanism
112 coagulation reaction determining mechanism
113 specimen buffer mechanism
114 centrifugal separating mechanism
115 control mechanism

**Claims**

1. A blood coagulation measuring device, **characterized by**:

   a measuring tube that holds a specimen in which whole blood and a blood coagulation accelerator are mixed with each other;
   a thermostatic device capable of holding the measuring tube at a constant temperature;
   a measurement light source capable of irradiating the measuring tube with a near infrared light of a specified wavelength range belonging to a range from 1050 nanometers to 1350 nanometers;
   a light receiving circuit unit that continuously measures an amount of light of the specified wavelength range transmitted through an inside of a specimen in the measuring tube; and
   an arithmetic processing device that sets a starting point to be when the blood coagulation accelerator is mixed and measures a time period until the amount of the light changes from increasing to decreasing.

2. The blood coagulation measuring device according to claim 1, **characterized in that** a wavelength of the specified wavelength range is in a range from 1250 nanometers to 1300 nanometers.

3. The blood coagulation measuring device according to claim 1, **characterized in that** the measurement light source is a single-wavelength LED light source with a peak wavelength in a range from 1050 nanometers to 1350 nanometers.

4. A method of determining blood coagulation reaction completion, **characterized by**:

   a process of irradiating a specimen obtained by mixing a blood coagulation accelerator and whole blood collected in a vessel, the specimen being irradiated with a near infrared light from a measurement light source;
   a process of measuring an intensity of light transmitted through an inside of the specimen, the intensity of the light being an amount of light of a specified wavelength range belonging to wavelengths from 1050 nanometers to 1350 nanometers; and
   a determining process of determining that a blood coagulation reaction is finished or not finished, based on information regarding an increase or decrease of the amount of the light during an arbitrary measurement.

5. The method according to claim 4, **characterized in that** a wavelength of the specified wavelength range is present in a range from 1250 nanometers to 1300 nanometers.

6. The method according to claim 4, **characterized in that** a peak wavelength of the measurement light source is a single-wavelength LED light source in a range from 1050 nanometers to 1350 nanometers.

7. The method according to any one of claims 4 to 6, **characterized in that** the determining process includes determining that the coagulation reaction is in progress when the amount of the light is increasing, and determining that the coagulation reaction is finished when the amount of the light is decreasing.

8. The method according to claim 7, **characterized in that** the determining process is a process of concurrently measuring transmitted light and scattered light intensities.

9. A method of determining blood coagulation reaction completion, **characterized by**:

   a process of irradiating a specimen obtained by mixing a blood coagulation accelerator and whole blood collected in a cylindrical vessel, the specimen being irradiated with a near infrared light from a measurement light source;
   a process of measuring an intensity of a transmitted light transmitted through an inside of the specimen, the intensity of the transmitted light being an amount of the transmitted light that is of a specified wavelength range belonging to wavelengths from 1050 nanometers to 1350 nanometers, the amount of the transmitted light being measured for each pixel, using an area sensor having a pixel area of h×w for the amount of the transmitted light; and
   determining that a blood coagulation reaction is finished or not finished, depending on a value of correlation coefficient r expressed by equation (2),
   (in equation (1), $\omega_i$ is a common logarithm of the transmitted light intensity $\mu_i$ at a pixel position i of an area sensor and in equation (2), $l_i$ is an optical path length of the measurement sample in the measuring tube at the pixel position i):

$$\omega_i = \log_{10} \mu_i \quad \dots (1)$$

$$r = \frac{\sum_{i=1}^{n}(l_i - \bar{l})(\omega_i - \bar{\omega})}{\sqrt{\sum_{i=1}^{n}(l_i - \bar{l})^2 \sum_{i=1}^{n}(\omega_i - \bar{\omega})^2}} \quad \dots (2)$$

10. The method according to claim 9, **characterized in that** when the correlation coefficient is 0.8 or less, the blood coagulation reaction is determined to be finished.

11. An automated blood centrifuge device, **characterized in** having:

    a centrifugal separating mechanism used in a laboratory test for performing centrifugal separation for a blood collection tube containing a measurement sample obtained by mixing whole blood and a blood coagulation accelerator;
    a measurement light source capable of irradiating the blood collection tube with a near infrared light of a specified wavelength range belonging to a range from 1050 nanometers to 1350 nanometers;
    a light intensity measuring mechanism that measures an amount of light of the specified wavelength range transmitted through an inside of a specimen in the blood collection tube;
    a coagulation reaction determining mechanism that determines that a coagulation reaction is in progress when the amount of the light exhibits an increasing trend and that determines that the coagulation reaction is finished when the amount of the light exhibits a decreasing trend; and
    a conveying mechanism that conveys, to the centrifugal separating mechanism, the blood collection tube for which the coagulation reaction is determined to be finished.

12. The automated blood centrifuge device according to claim 11, **characterized in that** a wavelength of the specified wavelength range is present in a range from 1250 nanometers to 1300 nanometers.

13. The automated blood centrifuge device according to claim 11, **characterized in that** a peak wavelength of the measurement light source is a LED light source of a single wavelength in a range from 1050 nanometers to 1350 nanometers.

**14.** The automated blood centrifuge device according to claim 11, **characterized in** further having:

a specimen buffer mechanism that temporarily holds the blood collection tube; and
a transporting mechanism that transports, to the specimen buffer mechanism, the blood collection tube for which the coagulation reaction is determined to be in progress by the coagulation reaction determining mechanism, and after waiting for a predetermined time, again, returns the blood collection tube to the light intensity measuring mechanism for measurement of an intensity of the light.

**15.** An automated blood centrifuge device, **characterized in** having:

a centrifugal separating mechanism for a laboratory test;
a mechanism that irradiates a blood collection tube with a near infrared light having a peak wavelength in a range from 1050 nanometers to 1350 nanometers;
a mechanism that measures, using an area sensor that has a pixel region of h×w, temporal variation of an intensity of a transmitted light of the near infrared light irradiated to the blood collection tube; and
a coagulation reaction determining mechanism that determines whether a blood coagulation reaction has finished, based on an amount of change of the intensity of the transmitted light per unit time, the coagulation reaction determining mechanism determining that the blood coagulation reaction has finished or has not finished, depending on a value of a correlation coefficient r expressed by equation (2), and having a conveying mechanism that conveys, to the centrifugal separating mechanism, the blood collection tube for which the blood coagulation reaction is determined to be finished,
(in equation (1), $\omega_i$ is a common logarithm of the transmitted light intensity $\mu_i$ at a pixel position i of an area sensor and in equation (2), $l_i$ is an optical path length of the measurement sample in the measuring tube at the pixel position i):

$$\omega_i = \log_{10} \mu_i \quad ... (1)$$

$$r = \frac{\sum_{i=1}^{n}\left(l_i - \bar{l}\right)(\omega_i - \bar{\omega})}{\sqrt{\sum_{i=1}^{n}\left(l_i - \bar{l}\right)^2 \sum_{i=1}^{n}(\omega_i - \bar{\omega})^2}} \quad ... (2)$$

**16.** The automated blood centrifuge device according to claim 15, **characterized in that** when the correlation coefficient is 0.8 or less, the blood coagulation reaction is determined to be finished.

# FIG.1

# FIG.2

# FIG.3

# FIG.4

# FIG.5

# FIG.6

# FIG.7

# FIG.8

EP 4 092 413 A1

# FIG.9

# FIG.10

# FIG.11

EP 4 092 413 A1

# FIG.12

```
                    ┌─────────┐
                    │  START  │
                    └────┬────┘
                         │
                         ▼
        ┌──────────────────────────┐
        │     LIGHT INTENSITY      │  ╭ S121
        │       MEASUREMENT        │
        └────────────┬─────────────┘
                     │
                     ▼
            ◇ COAGULATION ◇   ╭ S122
            ◇  REACTION  ◇ ─── NO ───▶  ◇ SPECIMEN BUFFER ◇  ╭ S124 ── REINJECT ──▶
            ◇ DETERMINATION ◇                                              │
                     │                          │                          │
                    YES  ╭ S123              ABNORMAL  ╭ S125              │
                     ▼                          ▼
        ┌──────────────────────────┐  ┌──────────────────────────┐
        │  CENTRIFUGAL SEPARATION  │  │  DISCHARGE AS ABNORMAL   │
        │                          │  │         SAMPLE           │
        └────────────┬─────────────┘  └────────────┬─────────────┘
                     │                               │
                     ▼                               │
                ┌─────────┐◀─────────────────────────┘
                │   END   │
                └─────────┘
```

# FIG.13

## INTERNATIONAL SEARCH REPORT

International application No.

PCT/JP2021/001540

### A. CLASSIFICATION OF SUBJECT MATTER

G01N 33/49(2006.01)i; G01N 21/59(2006.01)i
FI: G01N33/49 K; G01N21/59 H
According to International Patent Classification (IPC) or to both national classification and IPC

### B. FIELDS SEARCHED

Minimum documentation searched (classification system followed by classification symbols)
G01N21/59; G01N33/49

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | JP 2014-173904 A (SYSMEX CORPORATION) 22 September 2014 (2014-09-22) entire text all drawings | 1-16 |
| A | JP 2016-61585 A (GENIAL LIGHT, INC.) 25 April 2016 (2016-04-25) entire text all drawings | 1-16 |
| A | JP 2015-111123 A (HITACHI HIGH-TECHNOLOGIES CORP.) 18 June 2015 (2015-06-18) entire text all drawings | 1-16 |
| A | WO 2017/033562 A1 (HITACHI HIGH-TECHNOLOGIES CORP.) 02 March 2017 (2017-03-02) entire text all drawings | 1-16 |
| A | WO 2016/006362 A1 (HITACHI HIGH-TECHNOLOGIES CORP.) 14 January 2016 (2016-01-14) entire text all drawings | 1-16 |
| A | JP 2017-150966 A (NATIONAL INSTITUTE OF ADVANCED INDUSTRIAL SCIENCE AND TECHNOLOGY) 31 August 2017 (2017-08-31) entire text all drawings | 1-16 |

☒ Further documents are listed in the continuation of Box C.   ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 March 2021 (08.03.2021) | 16 March 2021 (16.03.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

25

**INTERNATIONAL SEARCH REPORT**

International application No.

PCT/JP2021/001540

C (Continuation). DOCUMENTS CONSIDERED TO BE RELEVANT

| Category* | Citation of document, with indication, where appropriate, of the relevant passages | Relevant to claim No. |
|---|---|---|
| A | JP 2016-191677 A (SYSMEX CORPORATION) 10 November 2016 (2016-11-10) entire text all drawings | 1-16 |
| A | US 2019/0033221 A1 (SHENZHEN THISTORY BIO-MEDICAL CO., LTD.) 31 January 2019 (2019-01-31) entire text all drawings | 1-16 |
| A | US 2012/0252127 A1 (SPECTRAL SCIENCES, INC.) 04 October 2012 (2012-10-04) entire text all drawings | 1-16 |

Form PCT/ISA/210 (continuation of second sheet) (January 2015)

**INTERNATIONAL SEARCH REPORT**
Information on patent family members

International application No.

PCT/JP2021/001540

| Patent Documents referred in the Report | Publication Date | Patent Family | Publication Date |
|---|---|---|---|
| JP 2014-173904 A | 22 Sep. 2014 | US 2014/0255254 A1 entire text all drawings EP 2775292 A1 CN 104034672 A | |
| JP 2016-61585 A | 25 Apr. 2016 | (Family: none) | |
| JP 2015-111123 A | 18 Jun. 2015 | US 2012/0282139 A1 entire text all drawings WO 2011/068049 A1 EP 2508891 A1 CN 102640004 A | |
| WO 2017/033562 A | 02 Mar. 2017 | US 2018/0231537 A1 entire text all drawings EP 3343207 A1 CN 107923853 A | |
| WO 2016/006362 A1 | 14 Jan. 2016 | CN 106471356 A entire text all drawings | |
| JP 2017-150966 A | 31 Aug. 2017 | (Family: none) | |
| JP 2016-191677 A | 10 Nov. 2016 | US 2016/0291040 A1 entire text all drawings EP 3091350 A1 CN 106018400 A | |
| US 2019/0033221 A1 | 31 Jan. 2019 | CN 107315094 A | |
| US 2012/0252127 A1 | 04 Oct. 2012 | (Family: none) | |

Form PCT/ISA/210 (patent family annex) (January 2015)

**EP 4 092 413 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014173904 A
- JP 2016061585 A
- JP 2015111123 A